# EUROPEAN PATENT SPECIFICATION

(11) **EP 4 337 645 B1**
(45) Date of publication and mention of the grant of the patent: **12.03.2025**
(21) Application number: 22726119.5
(22) Date of filing: 29.04.2022
(51) Int. Cl.: C07D 301/12, C07D 303/04

(54) **AN INTEGRATED PLANT AND AN INTEGRATED PROCESS FOR MAKING PROPENE OXIDE**
INTEGRIERTE ANLAGE UND INTEGRIERTES VERFAHREN ZUR HERSTELLUNG VON PROPENOXID
INSTALLATION INTÉGRÉE ET PROCÉDÉ INTÉGRÉ DE FABRICATION D'OXYDE DE PROPÈNE

(30) Priority: 10.05.2021 EP 21173006
(43) Date of publication of application: 20.03.2024
(73) Proprietor: Evonik Operations GmbH, 45128 Essen (DE); thyssenkrupp Uhde GmbH, 44141 Dortmund (DE)
(72) Inventor: BENJE, Michael, 65812 Bad Soden (DE); BRENDEL, Marc, 63486 Bruchköbel (DE); LODE, Florian, 61137 Schöneck (DE); BÄRZ, Manfred, 63579 Freigericht (DE)
(74) Representative: Evonik Patent Association
(86) International application number: PCT/EP2022/061456
(87) International publication number: WO 2022/238145

(56) References cited:
- EP-A1- 0 549 013
- EP-A1- 2 639 200
- WO-A1-02/085875
- WO-A1-2017/089074

## Description

### Field of the invention

The invention is directed at an integrated plant and an integrated process for making propene oxide.

### Background of the invention

A process for making propene oxide by epoxidation of propene with hydrogen peroxide in the presence of a titanium zeolite epoxidation catalyst is known from EP 0 100 119 A1.

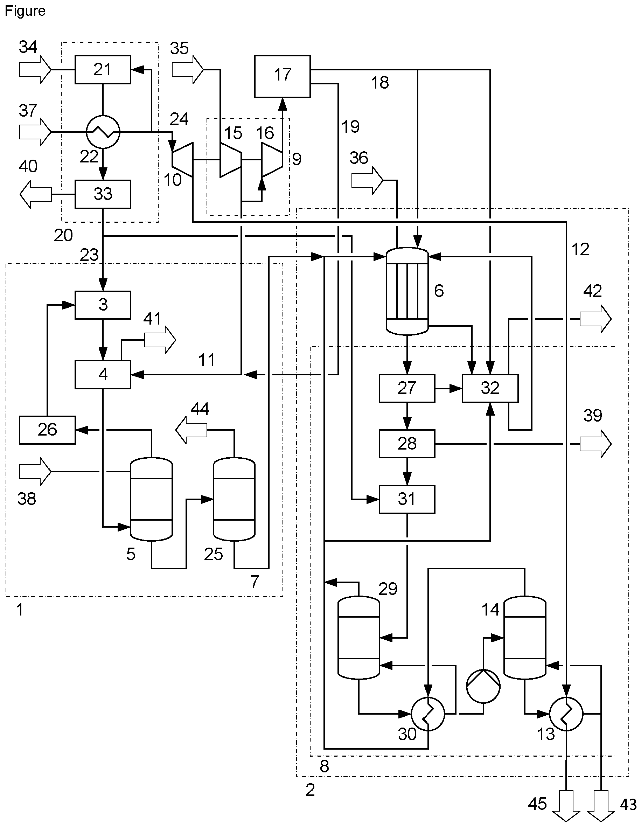

The most used process for producing hydrogen peroxide on an industrial scale is the anthraquinone process, which generates hydrogen peroxide by hydrogenating a working solution of an alkylanthraquinone or an alkyltetrahydroanthraquinone in a water immiscible solvent and oxidizing the hydrogenated solution with molecular oxygen, usually with air. The hydrogen peroxide is then extracted with water from the working solution and the working solution is reused for generating hydrogen peroxide. An overview of the anthraquinone process is given in Ullmann's Encyclopedia of Industrial Chemistry, online edition, Vol. A18, pages 397-409, DOI 0.1002/14356007.a13_443.pub2, and in particular in Fig. 5 on page 401. The hydrogenated solution is usually oxidized with pressurized air which has been compressed with an electrically driven compressor.

US 5,599,956 and WO 02/085875 disclose an integrated process for making propene oxide which combines propane dehydrogenation, hydrogen peroxide synthesis and propene epoxidation with hydrogen peroxide by using hydrogen produced by propane dehydrogenation for producing hydrogen peroxide, which is then reacted with propene provided by the propane dehydrogenation.

EP 549 013 disclose an integrated process for making propene oxide where a water-alcohol mixture, recovered from a reaction mixture of epoxidizing propene with hydrogen peroxide in an alcohol solvent, is used to extract hydrogen peroxide from the working solution of an anthraquinone process and the resulting extract is used as hydrogen peroxide source for epoxidizing propene.

WO 2017/089074 A1 and EP 2639200 A1 disclose an integrated process for the preparation of propylene oxide, wherein an anthraquinone process serves as a source for hydrogen peroxide.

### Summary of the invention

The inventor of the present invention has now found that integrating the process for making propene oxide from propene and hydrogen peroxide with an anthraquinone process by using a compressor driven by a backpressure steam turbine to provide pressurized air for oxidizing the hydrogenated solution in the anthraquinone process and using the steam leaving the backpressure steam turbine for heating one or more distillation columns of the work-up section of the unit for making propene oxide can save electrical energy compared to the prior art processes.

Subject of the invention is therefore an integrated plant for making propene oxide, comprising a unit for producing hydrogen peroxide by an anthraquinone process and a unit for making propene oxide from propene and hydrogen peroxide, the unit for producing hydrogen peroxide comprising
a) a hydrogenator for hydrogenating a working solution, containing an alkylanthraquinone, an alkyltetrahydroanthraquinone or both, with hydrogen,
b) an oxidizer for oxidizing hydrogenated working solution with an oxygen containing gas, and
c) an extraction column for extracting hydrogen peroxide from oxidized working solution,
   and the unit for making propene oxide from propene and hydrogen peroxide comprising
d) an epoxidation reactor with a feed conduit for hydrogen peroxide connected to the unit for producing hydrogen peroxide and a fixed bed of an epoxidation catalyst comprising a titanium zeolite, and
e) a work-up section for separating propene oxide and optionally solvent from an epoxidation reaction mixture,
characterized in that the integrated plant comprises an air compressor driven by a backpressure steam turbine, a conduit connecting an outlet of the air compressor with the oxidizer of the unit for producing hydrogen peroxide, and a conduit connecting the steam outlet of the backpressure steam turbine with a heat exchanger of a distillation column of the work-up section of the unit for making propene oxide.

Another subject of the invention is an integrated process for making propene oxide, comprising the steps
a) hydrogenating a working solution, containing an alkylanthraquinone, an alkyltetrahydroanthraquinone or both, with hydrogen in a hydrogenation reactor to provide a hydrogenated working solution comprising an alkylanthrahydroquinone, an alkyltetrahydroanthrahydroquinone or both,
b) oxidizing hydrogenated working solution obtained in step a) with an oxygen containing gas in an oxidation reactor to provide an oxidized working solution comprising hydrogen peroxide and an alkylanthraquinone, an alkyltetrahydroanthraquinone or both,
c) extracting hydrogen peroxide from oxidized working solution obtained in step b) to provide an aqueous solution of hydrogen peroxide,
d) reacting propene with the aqueous solution of hydrogen peroxide obtained in step c) in the presence of a titanium zeolite catalyst and a solvent to provide a reaction mixture comprising propene oxide, and
e) separating propene oxide and solvent from the reaction mixture obtained in step d) using at least one distillation column,
wherein air is compressed with an air compressor driven by a backpressure steam turbine, a part or all of the compressed air is passed to step b) to provide a part or all of said oxygen containing gas, and a part or all of the steam exiting the backpressure steam turbine is used to supply heat to a distillation column of step e).

### Brief description of drawings

The figure shows an embodiment of the invention with a steam reforming unit and an air separation unit integrated into the plant and the process of the invention.

### Detailed description of the invention

The integrated plant of the present invention comprises a unit (1) for producing hydrogen peroxide by an anthraquinone process and a unit (2) for making propene oxide from propene and hydrogen peroxide.

The unit (1) for producing hydrogen peroxide comprises a hydrogenator (3), an oxidizer (4) and an extraction column (5).

The hydrogenator (3) of the plant of the present invention is configured for hydrogenating a working solution which contains an alkylanthraquinone, an alkyltetrahydroanthraquinone or both, with hydrogen. The hydrogenator (3) of the plant of the invention may be of any type known from the prior art for hydrogenating a working solution comprising an alkylanthraquinone, an alkyltetrahydroanthraquinone or both. The hydrogenator (3) may comprise a bubble column reactor, a stirred-tank reactor, a tube reactor, a fixed-bed reactor, a loop reactor or a gas-lift reactor for carrying out the hydrogenation reaction, depending on whether a suspended hydrogenation catalyst or a fixed bed hydrogenation catalyst shall be used. The hydrogenator (3) preferably comprises a bubble column with a recycle and injection of hydrogen gas at the column bottom for use with a suspended catalyst as known from WO 2010/139728 and Ullmann's Encyclopedia of Industrial Chemistry, online edition, entry "Hydrogen Peroxide", DOI: 10.1002/14356007.a13_443.pub3, pages 13-14 and Fig. 8. The hydrogenator (3) preferably comprises a heat exchanger for removing the heat of reaction from the working solution, preferably a heat exchanger arranged inside the hydrogenation reactor. When a suspended hydrogenation catalyst shall be used, the hydrogenator (3) typically also comprises a separator for separating catalyst from the working solution and returning it to the hydrogenation reactor, such as a filter, which may operate by cross-flow filtration or dead-end filtration. The hydrogenator (3) may also comprise a hydrogen compressor for carrying out hydrogenation at a pressure higher than the pressure provided by the source of the hydrogen feed. The hydrogenator (3) may further comprise a separator for separating non-reacted hydrogen gas from the hydrogenated working solution and recycling it to the hydrogenation reactor. If such a separator is present, the hydrogenator (3) preferably also comprises a recycle compressor for recycling the non-reacted hydrogen gas.

The oxidizer (4) of the plant of the present invention is configured for oxidizing hydrogenated working solution with an oxygen containing gas. The oxidizer (4) comprises an oxidation reactor which may be of any type known from the prior art for oxidizing a hydrogenated working solution comprising an alkylanthrahydroquinone, an alkyltetrahydroanthrahydroquinone or both. Preferably, a bubble column, which is preferably operated in counter-current, is used as oxidation reactor. The bubble column can be free from internal devices, but preferably contains distribution devices in the form of packings or sieve plates, most preferably sieve plates in combination with internal heat exchangers. The oxidizer (4) preferably comprises a demister for separating droplets entrained in the off-gas leaving the oxidation reactor. The oxidizer (4) may further comprise a unit for recovering mechanical energy from off-gas leaving the oxidation reactor, such as a turboexpander as described in US 4,485,084 or a gas jet pump as described in WO 03/070632.

The extraction column (5) of the plant of the present invention is configured for extracting hydrogen peroxide from oxidized working solution. Any type of extraction column known from the prior art for extracting hydrogen peroxide with an aqueous extractant from oxidized working solution containing dissolved hydrogen peroxide may be used. The extraction column (5) is preferably a counter-current continuous extraction column, sieve-plate columns being most preferred. The plant of the present invention may additionally comprise a hydrogen peroxide purification unit for purifying the extracted aqueous hydrogen peroxide solution by removing working solution components, preferably a unit for washing the aqueous hydrogen peroxide solution with a solvent.

The unit (1) for producing hydrogen peroxide preferably also comprises a distillation unit (25) configured for concentrating an aqueous hydrogen peroxide solution. The distillation unit (25) typically comprises a hydrogen peroxide evaporator and a distillation column receiving vapor from the peroxide evaporator. Any type of hydrogen peroxide evaporator and distillation column known from the prior art for concentrating an aqueous hydrogen peroxide solution may be used. The hydrogen peroxide evaporator may be the distillation bottoms evaporator, which may be arranged separately from the distillation column or may be integrated into the distillation column, for example as disclosed in EP 0 419 406 A1, Fig. 4 or in EP 0 835 680 A1, Fig. 1 and 2. A separate thermosiphon evaporator passing a two-phase mixture of vapor and liquid to the distillation column may be used as distillation bottoms evaporator. The distillation unit (25) may also comprise both a hydrogen peroxide feed evaporator and a distillation bottoms evaporator, with compressed vapor being passed to the hydrogen peroxide feed evaporator, for example as disclosed in Fig. 1 and 2 of WO 2012/025333, or to the distillation bottoms evaporator or to both the hydrogen peroxide feed evaporator and the distillation bottoms evaporator. The distillation column may comprise trays or packings or a combination of both and preferably comprises structured packings to minimize pressure drop in the column. The distillation unit (25) may also comprise a vapor compressor receiving overhead vapor from the distillation column and passing compressed vapor as heating medium to the hydrogen peroxide evaporator. The vapor compressor may be a mechanical compressor, preferably a one stage mechanical compressor and is most preferably a water ring pump. The vapor compressor may alternatively be a gas jet pump and is preferably a steam driven ejector.

The unit (1) for producing hydrogen peroxide typically also comprises at least one buffer tank for storing aqueous hydrogen peroxide solution produced by the unit.

The unit (1) for producing hydrogen peroxide preferably further comprises a drier (26) for reducing the water content of the extracted working solution before recycling it to the hydrogenator (3). Any type of drier known from the prior art to be suitable for removing water from the working solution of an anthraquinone process may be used.

The unit (2) for making propene oxide comprises an epoxidation reactor (6) and a work-up section (8).

The epoxidation reactor (6) of the unit for making propene oxide is configured for reacting propene with hydrogen peroxide in the presence of a titanium zeolite catalyst. The epoxidation reactor (6) comprises a feed conduit (7) for hydrogen peroxide which is connected to the unit for producing hydrogen peroxide, preferably to a buffer tank as described in paragraph [014], and a fixed bed of an epoxidation catalyst which comprises a titanium zeolite. The epoxidation reactor (6) is preferably a fixed bed reactor configured for passing a mixture comprising propene, hydrogen peroxide and methanol over a fixed bed comprising a shaped titanium zeolite catalyst, preferably for passing the mixture over the catalyst fixed bed in downflow in a trickle bed mode. Preferably, a tube bundle reactor is used comprising fixed beds of the epoxidation catalyst within the tubes of the bundle. Such a tube bundle reactor preferably comprises from 5000 to 20000 parallel reaction tubes. The tube bundle reactor preferably has vertically arranged reaction tubes and comprises at least one distributor arranged above the entry of the reaction tubes, having openings for supplying liquid to each of the reaction tubes. The distributor preferably comprises separate openings for separately supplying two liquids to each of the reaction tubes, in particular for separately supplying a propene feed stream and a hydrogen peroxide feed stream to each of the reaction tubes. Suitable distributors are known from the prior art, for example from WO 2005/025716. This embodiment of the reactor is suitable for operating the process of the invention with trickle flow of liquid in the catalyst fixed bed. The epoxidation reactor (6) is preferably equipped with cooling means for cooling with a liquid cooling medium. Preferably, the epoxidation reactor (6) is a tube bundle reactor with a cooling jacket enclosing the reaction tubes. The cooling jacket preferably has a feed point for cooling medium near the entry of the reaction tubes, a withdrawal point for cooling medium near the end of the reaction tubes and at least one additional withdrawal point for cooling medium upstream of the withdrawal point for cooling medium near the end of the reaction tubes. Preferably, a tube bundle reactor as described in WO 2017/089076 is used.

The work-up section (8) of the unit (2) for making propene oxide is configured for separating propene oxide and optionally solvent from an epoxidation reaction mixture provided by the epoxidation reactor. The work-up section (8) comprises at least one distillation column (14) with a heat exchanger (13) supplying heat to the distillation column. The work-up section (8) preferably comprises units for separating and recovering non-reacted propene from the epoxidation reaction mixture, units for separating and purifying propene oxide and a unit for separating solvent used in the epoxidation reaction. The propene separation unit (27) for separating non-reacted propene from the epoxidation reaction mixture preferably comprises a flash evaporator and more preferably a series of flash evaporators and corresponding compressors as described in WO 2017/089079 on page 4, line 32 to page 6, line 3. The propene separation unit (27) preferably further comprises a propene rectifier column for separating the vapor provided by the flash evaporators into a liquid overhead propene stream, a bottoms stream containing higher boiling components and an overhead gas stream containing propene, oxygen and nitrogen added for inertisation. The propene rectifier is preferably also connected to the epoxidation reactor (6) for receiving an off-gas stream from the reactor. The propene oxide separation unit (28) preferably comprises a pre-separation column downstream of the unit for separating non-reacted propene, preferably a pre-separation column which has from 5 to 20 theoretical separation stages in the stripping section and less than 3 theoretical separation stages in a rectifying section. The pre-separation column receives the propene depleted reaction mixture provided in the propene separation unit (27). If the propene separation unit (27) comprises a propene rectifier, it also receives the bottoms product of the propene rectifier. The propene oxide separation unit (28) preferably further comprises a propene stripper receiving the overhead product of the pre-separation column and providing an overhead stream enriched in propene which, if a propene rectifier is used, is passed to the propene rectifier. The propene depleted bottoms product from the propene stripper is preferably passed to a propene oxide purification column which is preferably configured for purifying the feed by an extractive distillation, preferably an extractive distillation with addition of a compound containing an NH₂ group reactive to acetaldehyde as described in WO 2004/048355 and WO 2017/093204. The unit for separating the solvent used in the epoxidation reaction preferably comprises at least one distillation column (14) configured for providing an overhead product enriched in solvent. Preferably, the unit for separating the solvent comprises two heat integrated distillation columns (14, 29) in series, each with a bottoms evaporator (13, 30), configured for operating the second distillation column (14) at a higher pressure than the first distillation column (29) and for passing overhead vapor from the second distillation column (14) as heating medium to the bottoms evaporator (30) of the first distillation column (29). The unit for separating the solvent preferably receives the bottoms product from a pre-separation column as described above. If the propene oxide separation unit (28) comprises an extractive distillation, the unit for separating the solvent preferably also receives the bottoms product from the extractive distillation.

The work-up section of the unit for making propene oxide preferably also comprises a hydrogenation reactor (31) downstream of the unit (28) for separating propene oxide and upstream of the unit for separating the solvent. This hydrogenation reactor (31) preferably comprises a fixed bed containing a heterogeneous hydrogenation catalyst and is preferably configured for operation with downflow of liquid in a trickle flow mode. If the propene oxide separation unit (28) comprises a pre-separation column and an extractive distillation as described above, preferably the bottoms product from both these units is passed to the hydrogenation reactor (31).

The integrated plant of the present invention comprises an air compressor (9) which is driven by a backpressure steam turbine (10). A conduit (11) connects an outlet of the air compressor (9) with the oxidizer (4) of the unit (1) for producing hydrogen peroxide for supplying air as oxygen containing gas to the oxidizer (4). Another conduit (12) connects the steam outlet of the backpressure steam turbine (10) with a heat exchanger (13) of a distillation column (14) of the work-up section (8) of the unit (2) for making propene oxide for supplying heat with the steam exiting the backpressure steam turbine (10). This conduit (12) is preferably connected to a heating medium inlet of a bottoms evaporator (13) of the distillation column (14), preferably a bottoms evaporator of a distillation column (14) of a unit for separating solvent used in the epoxidation reaction. Most preferably, this conduit (12) is connected to a heating medium inlet of a bottoms evaporator (13) of the second distillation column (14) of a series of two heat integrated distillation columns (14, 29) as described further above.

The air compressor (9) may be any type known to be suitable for compressing air from ambient pressure to a pressure of from 0.11 to 1.6 MPa. The air compressor (9) may be a reciprocating compressor, a rotary screw compressor, a centrifugal compressor or an axial compressor and is preferably a centrifugal compressor. Preferably, a multistage compressor is used with coolers in between stages. The air compressor (9) is driven by a backpressure steam turbine (10) which is preferably directly connected to a drive shaft of the air compressor (9). An additional electric drive may also be connected to this drive shaft. The integrated plant may contain one or more additional air compressors with outlets connected to the oxidizer for supplying compressed air and these additional air compressors may be driven electrically or by additional steam turbines or by a combination of both.

The integrated plant preferably comprises a pressure control valve for adjusting the backpressure of the steam turbine (10), preferably a control valve upstream of the heat exchanger (13) receiving steam form the steam outlet of the backpressure steam turbine, the control valve being arranged in a steam conduit bypassing the steam turbine. The integrated plant preferably also comprises a flow control valve upstream of the heat exchanger (13) receiving steam form the steam outlet of the backpressure steam turbine.

The steam outlet of the backpressure steam turbine (10) may be connected to further equipment of the integrated plant in addition to the heat exchanger described above, such as additional heat exchangers of distillation columns or other equipment of the work-up section (8) of the unit (2) for making propene oxide.

In a preferred embodiment, the integrated plant of the invention comprises an additional air separation unit (17) and the air compressor (9) driven by a steam backpressure turbine (10) comprises two compression units in series, a first compression unit (15) and a second compression unit (16). The conduit (11) which connects an outlet of the air compressor (9) with the oxidizer (4) of the unit (1) for producing hydrogen peroxide is then connected to the first compression unit (15), and an outlet of the second compression unit (16) is connected to an inlet of the additional air separation unit (17). Any unit known to be suitable for separating air into an oxygen depleted gas and an oxygen enriched gas may be used for the air separation unit (17), such as a pressure swing adsorption unit, a membrane air separation unit or preferably a cryogenic air separation unit. The integrated plant then preferably comprises at least one conduit (18) connecting an outlet of the air separation unit (17) for oxygen depleted gas with the unit (2) for making propene oxide. The conduit (18) is preferably connected with a device of the unit (2) for making propene oxide where propene is condensed or absorbed into a liquid and oxygen may be enriched in a gas phase, such as a propene absorption unit (32) for absorbing separated propene with recovered solvent. The integrated plant may further comprise a conduit (19) which connects an outlet of the air separation unit (17) for gas enriched in oxygen with an inlet of the oxidizer (4) of the unit (1) for producing hydrogen peroxide. This embodiment provides the inert gas needed for preventing explosion risks in the unit (2) for making propene oxide with less consumption of electric energy than a conventional unit for producing nitrogen gas. The compression units (15, 16) may each be single stage or multiple stage compressors.

In another preferred embodiment, which may be combined with the previously described embodiments, the integrated plant of the invention comprises a steam reforming unit (20) for producing hydrogen from methane which comprises a steam reformer (21) and a steam generator (22) which is heated by product gas exiting the steam reformer (21). The steam reforming unit (20) typically also comprises a reactor for converting carbon monoxide to carbon dioxide by a water gas shift reaction, and a hydrogen separation unit (33) which separates hydrogen from carbon dioxide. A hydrogen outlet of the steam reforming unit (20) is connected by a conduit (23) with an inlet of the hydrogenator (3) of the unit (1) for producing hydrogen peroxide, and a steam outlet of the steam generator (22) is connected by a conduit (24) with an inlet of the backpressure steam turbine (10). This embodiment allows to use energy generated in the steam reformer (21) in the unit (2) for making propene oxide and thereby reduces the energy consumption of the integrated plant.

The integrated process of the invention for making propene oxide, which is preferably carried out in an integrated plant of the invention as described in the preceding paragraphs, comprises the steps of a) hydrogenating a working solution; b) oxidizing hydrogenated working solution obtained in step a); c) extracting hydrogen peroxide from oxidized working solution obtained in step b); d) reacting propene with the aqueous solution of hydrogen peroxide obtained in step c); and e) separating propene oxide and solvent from the reaction mixture obtained in step d).

In step a) of the integrated process of the invention, a working solution containing an alkylanthraquinone, an alkyltetrahydroanthraquinone or both is hydrogenated with hydrogen in a hydrogenation reactor to provide a hydrogenated working solution comprising an alkylanthrahydroquinone, an alkyltetrahydroanthrahydroquinone or both. The working solution preferably comprises one or more 2-alkylanthraquinones, 2-alkyltetrahydroanthraquinones or mixtures of both 2-alkylanthraquinones and 2-alkyltetrahydroanthraquinones, referred to as quinones in the following. The 2-alkylanthraquinone is preferably 2-ethylanthraquinone (EAQ), 2-amylanthraquinone (AAQ) or 2-(4-methylpentyl)-anthraquinone IHAQ and more preferably a mixture of EAQ with AAQ and/or IHAQ where the molar fraction of quinones carrying an ethyl group is from 0.05 to 0.95. The working solution preferably comprises both 2-alkylanthraquinones and the corresponding 2-alkyltetrahydroanthraquinones and the ratio of 2-alkyltetrahydroanthraquinones plus 2-alkyltetrahydroanthrahydroquinones to 2-alkylanthraquinones plus 2-alkylanthrahydroquinones is preferably maintained in the range of from 1 to 20 by adjusting the conditions of the hydrogenating step and of regenerating steps used in the anthraquinone process. The working solution also comprises at least one solvent for dissolving the quinone(s) and the hydroquinone(s). The working solution preferably comprises a mixture of alkylbenzenes having 9 or 10 carbon atoms as solvent for anthraquinones and at least one polar solvent selected from diisobutylcarbinol (DiBC), methylcyclohexylacetate (MCA), trioctylphosphate (TOP), tetrabutylurea (TBU) and N-octylcaprolactam as solvent for anthrahydroquinones. DiBC, MCA, TOP and TBU are preferred and TOP is most preferred.

In hydrogenation step a), all or a part of the quinones are converted to the corresponding hydroquinones. The hydrogenation is typically carried out in the presence of a heterogeneous hydrogenation catalyst. All hydrogenation catalysts known from the prior art for the anthraquinone cyclic process can be used as catalysts in the hydrogenation step. Noble metal catalysts containing palladium as the principal component are preferred. The catalysts can be used as a fixed bed catalyst or as a suspended catalyst and suspended catalysts can be either unsupported catalysts, such as palladium black, or supported catalysts, with suspended supported catalysts being preferred. SiO₂, TiO₂, Al₂O₃ and mixed oxides thereof, as well as zeolites, BaSO₄ or polysiloxanes, can be used as support materials for fixed-bed catalysts or supported suspended catalysts, with Al₂O₃ and sodium aluminum silicate being preferred. Catalysts in the form of monolithic or honeycombed moldings, the surface of which is coated with the noble metal, can also be used. Hydrogenation can be carried out in bubble column reactors, stirred-tank reactors, tube reactors, fixed-bed reactors, loop reactors or gas-lift reactors which can be equipped with devices for distributing hydrogen gas in the working solution, such as static mixers or injection nozzles. Preferably, a bubble column with a recycle and injection of hydrogen gas at the column bottom is used, such as described in WO 2010/139728 and in Ullmann's Encyclopedia of Industrial Chemistry, online edition, entry "Hydrogen Peroxide", DOI: 10.1002/14356007.a13_443.pub3, pages 13-14 and Fig. 8. Hydrogenation is preferably carried out at a temperature of from 20 to 100°C, more preferably 45 to 75°C, and a pressure of from 0.1 MPa to 1 MPa, more preferably 0.2 MPa to 0.5 MPa. The hydrogenation is preferably performed in such a way that most of the hydrogen introduced into the hydrogenation reactor, preferably more than 90 %, is consumed in a single pass through the reactor. The ratio between hydrogen and working solution fed to the hydrogenation reactor is preferably chosen to convert between 30 and 80 % of the quinones to the corresponding hydroquinones. If a mixture of 2-alkylanthraquinones and 2-alkyltetrahydroanthraquinones is used, the ratio between hydrogen and working solution is preferably chosen so that only the 2-alkyltetrahydroanthraquinones are converted to hydroquinones and the 2-alkylanthraquinones remain in the quinone form.

In step b) of the integrated process of the invention, hydrogenated working solution obtained in step a) is oxidized with an oxygen containing gas in an oxidation reactor to provide an oxidized working solution comprising hydrogen peroxide and an alkylanthraquinone, an alkyltetrahydroanthraquinone or both. The oxygen containing gas is preferably air or air enriched with oxygen. All oxidation reactors known from the prior art for the anthraquinone process can be used for the oxidation, bubble columns operated in counter-current being preferred. The bubble column can be free from internal devices, but preferably contains distribution devices in the form of packings or sieve plates, most preferably sieve plates in combination with internal coolers. Oxidation is preferably carried out at a temperature of from 30 to 70°C, more preferably from 40 to 60°C. Oxidation is preferably performed with an excess of oxygen to convert more than 90 %, preferably more than 95 %, of the hydroquinones to the quinone form.

In step c) of the integrated process of the invention, hydrogen peroxide is extracted from oxidized working solution obtained in step b) to provide an aqueous solution of hydrogen peroxide. The oxidized working solution of step b), which contains dissolved hydrogen peroxide, is extracted with an aqueous extractant to provide an aqueous hydrogen peroxide solution and an extracted oxidized working solution containing essentially no hydrogen peroxide. Deionized water, which may optionally contain additives for stabilizing hydrogen peroxide, for adjusting the pH and/or for corrosion protection, is preferably used for extracting the hydrogen peroxide. Preferably, phosphoric acid is added for adjusting the pH and for corrosion protection. Extraction is preferably carried out in a counter-current continuous extraction column, sieve-plate columns being most preferred. The aqueous hydrogen peroxide solution obtained by extraction may also be purified for removing working solution components, preferably by washing with a solvent, which is preferably a solvent comprised in the working solution.

The extracted oxidized working solution is typically recycled to step a) for operating a cyclic anthraquinone process. The extracted working solution of step c) is preferably dried before it is recycled to hydrogenating step a). Drying of the extracted working solution is preferably carried out by evaporating water from the working solution at a temperature of from 30 to 110°C, preferably 40 to 75 °C and a pressure of from 10 to 300 mbar, preferably 20 to 100 mbar. Such drying of extracted working solution at reduced pressure is preferably carried out as described in WO 03/070632 on page 8, line 24 to page 8, line 3.

The aqueous solution of hydrogen peroxide provided in extraction step c) is preferably concentrated in a distillation step to a concentration of from 45 to 90 % by weight hydrogen peroxide, preferably from 50 to 90 % by weight hydrogen peroxide. The hydrogen peroxide is concentrated at reduced pressure, preferably at a pressure of from 60 to 130 mbar, to prevent formation of explosive hydrogen peroxide vapors in the distillation.

In step d) of the integrated process of the invention, propene is reacted with the aqueous solution of hydrogen peroxide obtained in step c), optionally concentrated to a concentration of from 45 to 90 %, in the presence of a titanium zeolite catalyst and a solvent to provide a reaction mixture comprising propene oxide. Propene is preferably used in a molar excess to hydrogen peroxide, preferably at a molar ratio of propene to hydrogen peroxide of from 1.1:1 to 30:1, more preferably 2:1 to 10:1 and most preferably 3:1 to 5:1. In a preferred embodiment, propene is used in an excess sufficient to maintain an additional liquid phase rich in propene throughout step d). The propene feed may contain propane, preferably with a molar ratio of propane to propene of from 0.001 to 0.20 and more preferably of from 0.08 to 0.12.

The titanium zeolite catalyst preferably contains titanium atoms on silicon lattice positions. Preferably, a titanium silicalite catalyst is used, preferably with an MFI or MEL crystal structure. Most preferably a titanium silicalite 1 catalyst with MFI structure as known from EP 0 100 119 A1, is used. The titanium silicalite catalyst is preferably employed as a shaped catalyst in the form of granules, extrudates or shaped bodies. The shaped catalyst may contain 1 to 99 % of a binder or carrier material, all binders and carrier materials being suitable that do not react with hydrogen peroxide or with propene oxide under the reaction conditions employed for the epoxidation, silica being preferred as binder. Extrudates with a diameter of 1 to 5 mm are preferably used as shaped catalysts. The amount of catalyst employed may be varied within wide limits and is preferably chosen so that a hydrogen peroxide consumption of more than 90%, preferably more than 95%, is achieved within 1 minute to 5 hours under the employed epoxidation reaction conditions.

In step d), any solvent may be used which is not oxidized or is oxidized to only a small extent by hydrogen peroxide under the reaction conditions chosen and which dissolves in water at the reaction temperature in an amount of more than 10 % by weight. Solvents which are completely miscible with water are preferred. Suitable solvents are alcohols, such as methanol, ethanol, 2-propanol or tert-butanol; glycols, such as ethylene glycol, 1,2-propanediol or 1,3-propanediol; cyclic ethers, such tetrahydrofuran, dioxane or propylene oxide; glycol ethers, such ethylene glycol monomethyl ether, ethylene glycol monoethyl ether, ethylene glycol monobutyl ether or the propylene glycol monomethyl ethers; ketones, such as acetone or 2-butanone; and nitriles, such as acetonitrile and proprionitrile. Preferably, the solvent is methanol, 2-propanol or acetonitrile, and most preferably the solvent is a methanol solvent. The methanol solvent can be a technical grade methanol, a methanol solvent stream recovered in the work-up of the epoxidation reaction mixture or a mixture of both. The methanol solvent may comprise other solvents in minor amounts, such as ethanol, with the amount of such other solvents preferably being less than 2 % by weight. The solvent is preferably used in the epoxidation in a weight ratio of 0.5 to 20 relative to the combined weight of water and hydrogen peroxide.

The epoxidation reaction is preferably carried out at a temperature of 20 to 80°C, more preferably at 25 to 60°C. The pressure is preferably from 1.9 to 5.0 MPa, more preferably 2.1 to 3.6 MPa and most preferably 2.4 to 2.8 MPa. Propene is preferably used in an excess sufficient to maintain an additional liquid phase rich in propene throughout the epoxidation reaction. Using an excess of propene at a high pressure provides high reaction rate and hydrogen peroxide conversion and at the same time high selectivity for propene oxide. The epoxidation reaction is preferably carried out with addition of ammonia to improve epoxide selectivity as described in EP 0230949 A2. Ammonia is preferably added in an amount of from 100 to 3000 ppm based on the weight of hydrogen peroxide.

The epoxidation reaction is preferably carried out continuously, preferably in a fixed bed reactor by passing a mixture comprising propene, hydrogen peroxide and solvent over a fixed bed comprising a shaped titanium zeolite catalyst. The fixed bed reactor is preferably a tube bundle reactor and the catalyst fixed bed is arranged inside the reactor tubes. The fixed bed reactor is preferably equipped with cooling means and cooled with a liquid cooling medium. The temperature profile along the length of the catalyst fixed bed is preferably adjusted to keep the reaction temperature along 70 to 98 %, preferably along 80 to 95 %, of the length of the catalyst fixed bed within a range of less than 5 °C, preferably within a range of from 0.5 to 3 °C. The temperature of the cooling medium fed to the cooling means is preferably adjusted to a value 3 to 13 °C lower than the maximum temperature in the catalyst fixed bed. The epoxidation reaction mixture is preferably passed through the catalyst bed in down flow mode, preferably with a superficial velocity from 1 to 100 m/h, more preferably 5 to 50 m/h, most preferred 5 to 30 m/h. The superficial velocity is defined as the ratio of volume flow rate/cross section of the catalyst bed. Additionally it is preferred to pass the reaction mixture through the catalyst bed with a liquid hourly space velocity (LHSV) from 1 to 20 h⁻¹, preferably 1.3 to 15 h⁻¹. It is particularly preferred to maintain the catalyst bed in a trickle bed state during the epoxidation reaction. Suitable conditions for maintaining the trickle bed state during the epoxidation reaction are disclosed in WO 02/085873 on page 8 line 23 to page 9 line 15. The epoxidation reaction is most preferably carried out with a catalyst fixed bed maintained in a trickle bed state at a pressure close to the vapor pressure of propene at the reaction temperature, using an excess of propene that provides a reaction mixture comprising two liquid phases, a solvent rich phase and a propene rich liquid phase. Two or more fixed bed reactors may be operated in parallel or in series in order to be able to operate the epoxidation process continuously when regenerating the epoxidation catalyst. Regeneration of the epoxidation catalyst can be carried out by calcination, by treatment with a heated gas, preferably an oxygen containing gas or by a solvent wash, preferably by the periodic regeneration described in WO 2005/000827. Regeneration of the epoxidation catalyst is preferably carried out without removing it from the fixed bed reactor. Different methods of regeneration may be combined.

In step e) of the integrated process of the invention, propene oxide and solvent are separated from the reaction mixture obtained in step d) using at least one distillation column. The separation of propene oxide and solvent from the reaction mixture can be carried out by methods known from the prior art on epoxidizing olefins with hydrogen peroxide.

Preferably, the reaction mixture obtained in step d) is subjected to a pressure reduction and propene vapor formed by the pressure reduction is recompressed and cooled to recover propene by condensation. The pressure reduction is preferably carried out in several stages as described in WO 2017/089079 on page 4, line 32 to page 6, line 3. The compressed propene vapor is preferably fed to a propene distillation column and separated into an overhead product comprising non-reacted propene and a bottoms product containing compounds having a boiling point higher than propene, such as propene oxide and solvent. The overhead product comprising non-reacted propene can be recycled to the epoxidation reaction. The bottoms product can be combined with the liquid mixture remaining after the pressure reduction. The liquid mixture remaining after the pressure reduction is preferably separated by distillation in a pre-separation column to provide a crude propene oxide comprising propene oxide, solvent and residual propene as an overhead product and a solvent mixture comprising solvent, water and peroxides as a bottoms product. The pre-separation column is preferably operated to provide an overhead product comprising from 20 to 60 % of the solvent contained in the liquid phase of the last pressure reduction step. The pre-separation column preferably has from 5 to 20 theoretical separation stages in the stripping section and less than 3 theoretical separation stages in a rectifying section and is most preferably operated without reflux and without a rectifying section to minimize the residence time of propene oxide in the pre-separation column. The pre-separation column is preferably operated at a pressure of from 0.16 to 0.3 MPa. Propene oxide and solvent are condensed from the overhead product of the pre-separation column and propene is preferably stripped from the resulting condensate in a propene stripping column which provides a bottom stream comprising propene oxide and solvent which is essentially free of propene.

When a methanol solvent is used, a purified propene oxide is preferably separated from the bottoms stream of the propene stripping column in an extractive distillation using water as the extraction solvent. The extractive distillation is preferably operated with additional feeding of a reactive compound containing an unsubstituted NH₂ group and capable of reacting with acetaldehyde during the extractive distillation, as described in WO 2004/048335. Extractive distillation with a reactive compound provides a high purity propene oxide containing less than 50 ppm of carbonyl compounds.

The solvent mixture comprising solvent, water and peroxides, which is obtained after separating non-reacted propene and propene oxide product, is preferably subjected to a catalytic hydrogenation for hydrogenating the peroxides contained in the solvent mixture. The catalytic hydrogenation is preferably carried out to a conversion of hydrogen peroxide that provides a hydrogenated solvent mixture containing less than 0.1 % by weight hydrogen peroxide. The reaction conditions of this catalytic hydrogenation are preferably selected to provide a hydrogenated solvent mixture comprising from 1 to 1000 mg/kg of acetaldehyde. The catalytic hydrogenation is preferably carried out at a hydrogen partial pressure of from 0.5 to 30 MPa, more preferably of from 1 to 25 MPa and most preferably of from 1 to 5 MPa. The temperature is preferably in the range of from 80 to 180 °C, more preferably from 90 to 150 °C. The catalytic hydrogenation is carried out in the presence of a hydrogenation catalyst, preferably a heterogeneous hydrogenation catalyst. Raney nickel and Raney cobalt may be used as hydrogenation catalyst. Preferably, a supported metal catalyst comprising one or more of metals selected from the group consisting of Ru, Rh, Pd, Pt, Ag, Ir, Fe, Cu, Ni and Co on a catalyst support is used. The metal is preferably platinum, palladium, iridium, ruthenium or nickel and most preferably ruthenium or nickel. The catalyst support can be any solid which is inert and does not deteriorate under the hydrogenation conditions. Suitable as catalyst support are activated carbon, the oxides SiO₂, TiO₂, ZrO₂ and Al₂O₃, and mixed oxides comprising at least two of silicon, aluminum, titanium and zirconium. SiO₂, Al₂O₃ and mixed oxides of silicon and aluminum are preferably used as the catalyst support for the supported metal catalyst. The catalyst support is preferably shaped as spheres, pellets, tablets, granules or extrudates. Preferred are extrudates with a diameter of from 0.5 to 5mm, especially from 1 to 3 mm, and a length of from 1 to 10 mm. The supported metal catalyst preferably comprises from 0.01 to 60 wt.% metal. Supported noble metal catalysts preferably comprise from 0.1 to 5 % metal. Supported nickel and cobalt catalysts preferably comprise from 10 to 60 % metal. The supported metal catalyst may be prepared by methods known in the art, preferably by impregnating the catalyst support with a metal salt followed by reducing the metal salt to the catalytically active metal. Suitable supported metal catalysts are commercially available, for example from Clariant under the NISAT^{®} trade name and from Evonik Industries under the Octolyst^{®} trade name.

After separation of non-reacted propene and propene oxide product from the reaction mixture of step d) and optionally hydrogenating as described in the previous paragraph, the resulting mixture containing solvent and water is preferably separated in at least one distillation stage to provide a recovered solvent as an overhead product. Preferably, the solvent mixture is separated in two subsequent distillation stages providing recovered solvent as an overhead product from both stages. The two distillation stages are preferably operated with a higher pressure in the second stage and overhead product vapor from the second stage is used for heating the bottoms evaporator of the first stage in order to save energy. An acid may be added prior to or during the distillation steps to reduce the content of volatile organic amines in the recovered solvent as described in WO 2004/048354 on page 7, line 25 to page 8, line 22 and in WO 2018/206505 on page 6, line 35 to page 7, line 29.

In the integrated process of the invention, air is compressed with an air compressor which is driven by a backpressure steam turbine, a part or all of the compressed air is passed to step b) to provide a part or all of said oxygen containing gas, and a part or all of the steam exiting the backpressure steam turbine is used to supply heat to a distillation column of step e). Any air compressor described above in paragraph [020] may be used. The air is preferably compressed to a pressure of from 0.11 to 1.6 MPa, more preferably from 0.30 to 0.60 MPa, prior to passing it to step b). The compressed air is preferably cooled to a temperature in the range from 20 °C to 60 °C before introducing it into the oxidation reactor of step b). Using an air compressor driven by a backpressure steam turbine for providing air to the oxidation step of the anthraquinone process significantly reduces the consumption of electric energy in the integrated process compared to a typical anthraquinone process using air compressed with an electrically driven air compressor.

Steam exiting the backpressure steam turbine may be used to supply heat to one or several distillation columns used in the distillation steps described further above in paragraphs [041], [042] and [044]. Preferably, the solvent used in step d) is selected from methanol, 2-propanol and acetonitrile and the steam exiting the backpressure steam turbine is used to supply heat to a distillation column separating the solvent as an overhead product, preferably supplying heat to a column reboiler. A part of the steam exiting the backpressure steam turbine may also be used in other steps of the integrated process, such as for supplying heat to a distillation unit for concentrating the extracted hydrogen peroxide. The backpressure steam turbine is preferably operated at a backpressure of from 0.6 to 4.0 MPa to provide steam having a temperature in the range of from 160 °C to 260 °C.

In a preferred embodiment of the process of the invention, the air compressor is a two-stage compressor, compressed air from the first compression unit is passed as oxygen containing gas to step b) and compressed air from the second compression unit is passed to an air separation unit which provides a gas stream enriched in nitrogen and a gas stream enriched in oxygen. All or a part of the gas stream enriched in nitrogen is then passed to step d) or to step e) or to both steps d) and e) to prevent formation of flammable gas mixtures. Preferably, parts of the gas stream enriched in nitrogen are introduced into the process units where propene is condensed or absorbed into a liquid and oxygen may be enriched in a gas phase.

The gas stream enriched in oxygen provided by the air separation unit may be advantageously used in different ways. In a first embodiment, all or a part of the gas stream enriched in oxygen is passed to step b) to provide oxygen containing gas, which increases productivity of the oxidizing step and reduces the size of the oxidation reactor. In a second embodiment, which may be combined with the first embodiment, all or a part of the gas stream enriched in oxygen is used to oxidize an off-gas generated in step d) or in step e) or off-gases of both steps d) and e). In a third embodiment, which may be combined with the first and/or the second embodiment, all or a part of the gas stream enriched in oxygen is used to oxidize a waste water generated in step e). In this embodiment, gas stream enriched in oxygen may be used in a wet oxidation process carried out with heating or may be used to supply oxygen to an aerobic biological wastewater treatment plant.

In another preferred embodiment, the process of the invention comprises the additional steps of f) reacting natural gas or methane with water in the presence of a steam reforming catalyst and g) separating hydrogen from the reaction mixture provided in step f). Heat is recovered from the reaction mixture in step f) in a steam generator, the steam generated in step f) is used to drive the backpressure steam turbine and hydrogen separated in step g) is passed to step a). Step f) is typically carried out with natural gas as the methane source. Suitable steam reforming catalysts and devices for methane steam reforming with heat recovery in a steam generator are known from the prior art. Suitable methods and devices for separating hydrogen from the reaction mixture of a steam reformer are also known from the prior art. Step g) preferably comprises purifying hydrogen by pressure swing adsorption to remove carbon monoxide and sulfur compounds and provide a hydrogen gas quality suitable for the hydrogenating step a). This embodiment improves the overall energy efficiency of the process.

In yet another embodiment, steam generated in a propane dehydrogenation unit supplying propene to the unit for making propene oxide from propene and hydrogen peroxide may be used for driving the backpressure steam turbine.

The figure illustrates the invention with an embodiment additionally comprising a steam reforming unit (20) and an air separation unit (17) integrated into the plant and the process of the invention. In this embodiment, the epoxidation reactor (6) is a tube bundle reactor configured for trickle flow operation and oxygen depleted gas from the air separation unit (17) is passed to the epoxidation reactor (6) to prevent enrichment of oxygen in the gas phase present in the reactor. The embodiment shown in the figure is a process using solvent in the epoxidation step and comprises a propene absorption unit (32) for absorbing separated propene into recovered solvent. A purge gas stream from the epoxidation reactor (6) is passed to the propene absorption unit (32) and the residual gas remaining after absorbing propene into the solvent is purged from the propene absorption unit (32). The solvent into which the recovered propene has been absorbed is recycled to the epoxidation reactor (6).

### Examples:

Comparison calculation for an integrated plant with and without an air compressor driven by a backpressure steam turbine, a conduit connecting an outlet of the air compressor with the oxidizer of the unit for producing hydrogen peroxide, and a conduit connecting the steam outlet of the backpressure steam turbine with a heat exchanger of a distillation column of the work-up section of the unit for making propene oxide.

Basic assumption: An integrated plant for making propene oxide, comprising a unit (1) for producing hydrogen peroxide by an anthraquinone process and a unit (2) for making propene oxide from propene and hydrogen peroxide, the unit (1) for producing hydrogen peroxide comprising a hydrogenator (3) for hydrogenating a working solution, containing an alkylanthraquinone, an alkyltetrahydroanthraquinone or both, with hydrogen, an oxidizer (4) for oxidizing hydrogenated working solution with an oxygen containing gas, and an extraction column (5) for extracting hydrogen peroxide from oxidized working solution, and the unit (2) for making propene oxide from propene and hydrogen peroxide comprising an epoxidation reactor (6) with a feed conduit (7) for hydrogen peroxide connected to the unit (1) for producing hydrogen peroxide and a fixed bed of an epoxidation catalyst comprising a titanium zeolite, and a work-up section (8) for separating propene oxide and optionally solvent from an epoxidation reaction mixture. Wherein the unit (2) for making propene oxide from propene and hydrogen peroxide requires an amount of 93.8 t/h steam, supplied at 20.5 bar absolute and 219°C, denoted as medium pressure steam (MPS).

Further basic assumption: sufficient high-pressure steam (HPS) is available at a pressure of 85 bar absolute and 520°C. For injection sufficient steam condensate is available at a pressure of 1.3 bar absolute and 101°C.

Case 1 - Pressure reduction via condensate injection: Pressure of available high-pressure steam (HPS) from steam source is reduced to required steam supply conditions of unit 1 (MPS) by injection of condensate. With above basic assumptions, an amount of 74.2 t/h HPS and 19.5 t/h condensate are required to achieve an amount of 93.8 t/h MPS at 20.5 bar absolute and 219°C supplied to unit 1.

Case 2 - Utilization of high-pressure steam to drive air compressor (9): Pressure of available high pressure steam (HPS) from steam source is reduced by using said steam to drive the turbine (10) of the air compressor (9), which is connected as claimed in claim 1 and 7. With the assumption of an isentropic coefficient of 60% and a set outlet pressure of 20.5 bar absolute, a generated power equivalent "at shaft" of 5.7 MW and a steam temperature of 375°C at the outlet of the turbine result. The steam conditions present at outlet of compressor turbine are adjusted to required steam supply conditions of the unit (2) for making propene oxide from propene and hydrogen peroxide (MPS) by injection of condensate. With above summarized basic assumptions, an amount of 81.0 t/h HPS and 12.8 t/h condensate are required to achieve an amount of 93.8 t/h MPS at 20.5 bar absolute and 219°C supplied to unit (2) for making propene oxide from propene and hydrogen peroxide

A comparison between case 1 and case 2 yields an HPS steam demand being 6.8 t/h higher for case 2 compared to case 1, due to the smaller benefit by condensate injection. However, case 2 results in a power uptake "at shaft" of 5.7 MW and corresponding saving of electrical energy compared to case 1.

The saving of electrical energy at the cost of increased HPS consumption leads to major savings over a wide range of steam and electricity cost assumptions.

### List of reference signs:

- 1: Unit for producing hydrogen peroxide
- 2: Unit for making propene oxide
- 3: Hydrogenator
- 4: Oxidizer
- 5: Extraction column
- 6: Epoxidation reactor
- 7: Feed conduit for hydrogen peroxide
- 8: Work-up section for separating propene oxide and optionally solvent
- 9: Air compressor
- 10: Backpressure steam turbine
- 11: Conduit connecting the air compressor (9) with the oxidizer (4)
- 12: Conduit connecting the backpressure steam turbine (10) with a heat exchanger (13)
- 13: Heat exchanger of a distillation column (14)
- 14: Distillation column of the work-up section (8) (second distillation column)
- 15: First compression unit
- 16: Second compression unit
- 17: Air separation unit
- 18: Conduit connecting the air separation unit (17) with the unit (2) for making propene oxide
- 19: Conduit connecting the air separation unit (17) with the oxidizer (4)
- 20: Steam reforming unit
- 21: Steam reformer
- 22: Steam generator
- 23: Conduit connecting the steam reforming unit (20) with the hydrogenator (3)
- 24: Conduit connecting the steam generator (22) with the backpressure steam turbine (10)
- 25: Distillation unit for concentrating aqueous hydrogen peroxide solution
- 26: Drier
- 27: Propene separation unit
- 28: Propene oxide separation unit
- 29: First distillation column
- 30: Bottoms evaporator of the first distillation column (29)
- 31: Hydrogenation reactor of the work-up section (8)
- 32: Propene absorption unit
- 33: Hydrogen separation unit
- 34: Natural gas to steam reformer (21)
- 35: Air to air compressor (9)
- 36: Propene to epoxidation reactor (6)
- 37: Water to steam generator (22)
- 38: Water to extraction column (5)
- 39: Propene oxide from propene oxide separation unit (28)
- 40: Carbon dioxide from hydrogen separation unit (33)
- 41: Oxidizer off-gas from oxidizer (4)
- 42: Off-gas from propene absorption unit (32)
- 43: Aqueous effluent from work-up section (8)
- 44: Water from distillation unit (25)
- 45: Steam condensate from heat exchanger (13)

## Claims

1. An integrated plant for making propene oxide, comprising a unit (1) for producing hydrogen peroxide by an anthraquinone process and a unit (2) for making propene oxide from propene and hydrogen peroxide, the unit (1) for producing hydrogen peroxide comprising
a) a hydrogenator (3) for hydrogenating a working solution, containing an alkylanthraquinone, an alkyltetrahydroanthraquinone or both, with hydrogen,
b) an oxidizer (4) for oxidizing hydrogenated working solution with an oxygen containing gas, and
c) an extraction column (5) for extracting hydrogen peroxide from oxidized working solution,
and the unit (2) for making propene oxide from propene and hydrogen peroxide comprising
d) an epoxidation reactor (6) with a feed conduit (7) for hydrogen peroxide connected to the unit (1) for producing hydrogen peroxide and a fixed bed of an epoxidation catalyst comprising a titanium zeolite, and
e) a work-up section (8) for separating propene oxide and optionally solvent from an epoxidation reaction mixture,
**characterized in that** the integrated plant comprises an air compressor (9) driven by a backpressure steam turbine (10), a conduit (11) connecting an outlet of the air compressor (9) with the oxidizer (4) of the unit (1) for producing hydrogen peroxide, and a conduit (12) connecting the steam outlet of the backpressure steam turbine (10) with a heat exchanger (13) of a distillation column (14) of the work-up section (8) of the unit (2) for making propene oxide.

2. The integrated plant of claim 1, wherein the air compressor (9) comprises a first compression unit (15) and a second compression unit (16), said conduit (11) connecting an outlet of the air compressor (9) with the oxidizer (4) of the unit (1) for producing hydrogen peroxide is connected to the first compression unit (15), and an outlet of the second compression unit (16) is connected to an inlet of an additional air separation unit (17).

3. The integrated plant of claim 2, comprising a conduit (18) connecting an outlet of the air separation unit (17) for oxygen depleted gas with the unit (2) for making propene oxide.

4. The integrated plant of claim 2 or 3, comprising a conduit (19) connecting an outlet of the air separation unit (17) for gas enriched in oxygen with an inlet of the oxidizer (4) of the unit (1) for producing hydrogen peroxide.

5. The integrated plant of any one of claims 2 to 4, wherein the air separation unit (17) comprises a cryogenic air separation unit.

6. The integrated plant of any one of claims 1 to 5, additionally comprising a steam reforming unit (20) for producing hydrogen from methane, the steam reforming unit (20) comprising a steam reformer (21) and a steam generator (22) heated by product gas exiting the steam reformer (21), a conduit (23) connecting a hydrogen outlet of the steam reforming unit (20) with an inlet of the hydrogenator (3) of the unit (1) for producing hydrogen peroxide, and a conduit (24) connecting a steam outlet of the steam generator (22) with an inlet of the backpressure steam turbine (10).

7. An integrated process for making propene oxide, comprising the steps
a) hydrogenating a working solution, containing an alkylanthraquinone, an alkyltetrahydroanthraquinone or both, with hydrogen in a hydrogenation reactor to provide a hydrogenated working solution comprising an alkylanthrahydroquinone, an alkyltetrahydroanthrahydroquinone or both,
b) oxidizing hydrogenated working solution obtained in step a) with an oxygen containing gas in an oxidation reactor to provide an oxidized working solution comprising hydrogen peroxide and an alkylanthraquinone, an alkyltetrahydroanthraquinone or both,
c) extracting hydrogen peroxide from oxidized working solution obtained in step b) to provide an aqueous solution of hydrogen peroxide,
d) reacting propene with the aqueous solution of hydrogen peroxide obtained in step c) in the presence of a titanium zeolite catalyst and a solvent to provide a reaction mixture comprising propene oxide, and
e) separating propene oxide and solvent from the reaction mixture obtained in step d) using at least one distillation column,
**characterized in that** air is compressed with an air compressor driven by a backpressure steam turbine, a part or all of the compressed air is passed to step b) to provide a part or all of said oxygen containing gas, and a part or all of the steam exiting the backpressure steam turbine is used to supply heat to a distillation column of step e).

8. The process of claim 7, wherein the air compressor is a two-stage compressor, compressed air from the first compression unit is passed as oxygen containing gas to step b), compressed air from the second compression unit is passed to an air separation unit providing a gas stream enriched in nitrogen and a gas stream enriched in oxygen, and all or a part of the gas stream enriched in nitrogen is passed to step d) or to step e) or to both steps d) and e) to prevent formation of flammable gas mixtures.

9. The process of claim 8, wherein all or a part of the gas stream enriched in oxygen is passed to step b) to provide oxygen containing gas.

10. The process of claim 8, wherein all or a part of the gas stream enriched in oxygen is used to oxidize an off-gas generated in step d) or in step e) or off-gases of both steps d) and e).

11. The process of claim 8, wherein all or a part of the gas stream enriched in oxygen is used to oxidize a waste water generated in step e).

12. The process of any one of claims 7 to 11, wherein the backpressure steam turbine is operated at a backpressure of from 0.6 to 4.0 MPa.

13. The process of any one of claims 7 to 12, additionally comprising the steps of
f) reacting natural gas or methane with water in the presence of a steam reforming catalyst with recovery of heat in a steam generator, providing a reaction mixture comprising hydrogen, and
g) separating hydrogen from the reaction mixture of step f),
wherein steam generated in step f) is used to drive said backpressure steam turbine and hydrogen separated in step g) is passed to step a).

14. The process of any one of claims 7 to 13, wherein the solvent used in step d) is selected from methanol, 2-propanol and acetonitrile and the steam exiting the backpressure steam turbine is used to supply heat to a distillation column separating the solvent as an overhead product.

## Patentansprüche

1. Integrierte Anlage zur Herstellung von Propenoxid, umfassend eine Einheit (1) zur Herstellung von Wasserstoffperoxid durch ein Anthrachinonverfahren und eine Einheit (2) zur Herstellung von Propenoxid aus Propen und Wasserstoffperoxid, wobei die Einheit (1) zur Herstellung von Wasserstoffperoxid umfasst:
a) eine Hydrieranlage (3) zum Hydrieren einer Arbeitslösung, die ein Alkylanthrachinon, ein Alkyltetrahydroanthrachinon oder beides enthält, mit Wasserstoff,
b) eine Oxidationsanlage (4) zum Oxidieren von hydrierter Arbeitslösung mit einem sauerstoffhaltigen Gas, und
c) eine Extraktionskolonne (5) zum Extrahieren von Wasserstoffperoxid aus oxidierter Arbeitslösung,
und die Einheit (2) zur Herstellung von Propenoxid aus Propen und Wasserstoffperoxid, umfassend
d) einen Epoxidationsreaktor (6) mit einer Zuleitung (7) für Wasserstoffperoxid, die mit der Einheit (1) zur Herstellung von Wasserstoffperoxid verbunden ist, und einem Festbett eines Epoxidationskatalysators, der einen Titanzeolith umfasst, und
e) einen Aufarbeitungsabschnitt (8) zum Abtrennen von Propenoxid und gegebenenfalls Lösungsmittel aus einem Epoxidationsreaktionsgemisch,
**dadurch gekennzeichnet, dass** die integrierte Anlage einen von einer Gegendruck-Dampfturbine (10) angetriebenen Luftkompressor (9), eine Leitung (11), die einen Auslass des Luftkompressors (9) mit der Oxidationsanlage (4) der Einheit (1) zur Herstellung von Wasserstoffperoxid verbindet, und eine Leitung (12), die den Dampfauslass der Gegendruck-Dampfturbine (10) mit einem Wärmetauscher (13) einer Destillationskolonne (14) des Aufarbeitungsabschnitts (8) der Einheit (2) zur Herstellung von Propenoxid verbindet, umfasst.

2. Integrierte Anlage nach Anspruch 1, wobei der Luftkompressor (9) eine erste Kompressionseinheit (15) und eine zweite Kompressionseinheit (16) umfasst, die Leitung (11), die einen Auslass des Luftkompressors (9) mit der Oxidationsanlage (4) der Einheit (1) zur Herstellung von Wasserstoffperoxid verbindet, mit der ersten Kompressionseinheit (15) verbunden ist und ein Auslass der zweiten Kompressionseinheit (16) mit einem Einlass einer zusätzlichen Lufttrenneinheit (17) verbunden ist.

3. Integrierte Anlage nach Anspruch 2, umfassend eine Leitung (18), die einen Auslass der Lufttrenneinheit (17) für sauerstoffarmes Gas mit der Einheit (2) zur Herstellung von Propenoxid verbindet.

4. Integrierte Anlage nach Anspruch 2 oder 3, umfassend eine Leitung (19), die einen Auslass der Lufttrenneinheit (17) für mit Sauerstoff angereichertes Gas mit einem Einlass der Oxidationsanlage (4) der Einheit (1) zur Herstellung von Wasserstoffperoxid verbindet.

5. Integrierte Anlage nach einem der Ansprüche 2 bis 4, wobei die Lufttrenneinheit (17) eine kryogene Lufttrenneinheit umfasst.

6. Integrierte Anlage nach einem der Ansprüche 1 bis 5, zusätzlich umfassend eine Dampfreformierungseinheit (20) zur Herstellung von Wasserstoff aus Methan, wobei die Dampfreformierungseinheit (20) einen Dampfreformer (21) und einen Dampferzeuger (22), der durch aus dem Dampfreformer (21) austretendes Produktgas beheizt wird, eine Leitung (23), die einen Wasserstoffauslass der Dampfreformierungseinheit (20) mit einem Einlass der Hydrieranlage (3) der Einheit (1) zur Herstellung von Wasserstoffperoxid verbindet, und eine Leitung (24), die einen Dampfauslass des Dampferzeugers (22) mit einem Einlass der Gegendruck-Dampfturbine (10) verbindet, umfasst.

7. Integriertes Verfahren zur Herstellung von Propenoxid, umfassend die Schritte
a) Hydrieren einer Arbeitslösung, die ein Alkylanthrachinon, ein Alkyltetrahydroanthrachinon oder beides enthält, mit Wasserstoff in einem Hydrierungsreaktor, um eine hydrierte Arbeitslösung bereitzustellen, die ein Alkylanthrahydrochinon, ein Alkyltetrahydroanthrahydrochinon oder beides umfasst,
b) Oxidieren der bei Schritt a) erhaltenen hydrierten Arbeitslösung mit einem sauerstoffhaltigen Gas in einem Oxidationsreaktor, um eine oxidierte Arbeitslösung bereitzustellen, die Wasserstoffperoxid und ein Alkylanthrachinon, ein Alkyltetrahydroanthrachinon oder beides umfasst,
c) Extrahieren von Wasserstoffperoxid aus bei Schritt b) erhaltener oxidierter Arbeitslösung, um eine wässrige Lösung von Wasserstoffperoxid bereitzustellen,
d) Umsetzen von Propen mit der bei Schritt c) erhaltenen wässrigen Lösung von Wasserstoffperoxid in Gegenwart eines Titanzeolith-Katalysators und eines Lösungsmittels, um ein Reaktionsgemisch, das Propenoxid umfasst, bereitzustellen, und
e) Abtrennen von Propenoxid und Lösungsmittel aus dem bei Schritt d) erhaltenen Reaktionsgemisch unter Verwendung wenigstens einer Destillationskolonne,
**dadurch gekennzeichnet, dass** Luft mit einem Luftkompressor komprimiert wird, der von einer Gegendruck-Dampfturbine angetrieben wird, ein Teil der oder die gesamte komprimierte Luft zu Schritt b) geleitet wird, um einen Teil oder das gesamte des sauerstoffhaltigen Gases bereitzustellen, und ein Teil des oder der gesamte Dampf, der aus der Gegendruck-Dampfturbine austritt, verwendet wird, um einer Destillationskolonne von Schritt e) Wärme zuzuführen.

8. Verfahren nach Anspruch 7, wobei der Luftkompressor ein zweistufiger Kompressor ist, komprimierte Luft von der ersten Kompressionseinheit als sauerstoffhaltiges Gas zu Schritt b) geleitet wird, komprimierte Luft von der zweiten Kompressionseinheit zu einer Lufttrenneinheit geleitet wird, die einen mit Stickstoff angereicherten Gasstrom und einen mit Sauerstoff angereicherten Gasstrom bereitstellt, und der gesamte oder ein Teil des mit Stickstoff angereicherten Gasstroms zu Schritt d) oder Schritt e) oder beiden Schritten d) und e) geleitet wird, um die Entstehung brennbarer Gasgemische zu verhindern.

9. Verfahren nach Anspruch 8, wobei der gesamte oder ein Teil des mit Sauerstoff angereicherten Gasstroms zu Schritt b) geleitet wird, um sauerstoffhaltiges Gas bereitzustellen.

10. Verfahren nach Anspruch 8, wobei der gesamte oder ein Teil des mit Sauerstoff angereicherten Gasstroms zum Oxidieren eines bei Schritt d) oder bei Schritt e) erzeugten Abgases oder Abgase beider Schritte d) und e) verwendet wird.

11. Verfahren nach Anspruch 8, wobei der gesamte oder ein Teil des mit Sauerstoff angereicherten Gasstroms zum Oxidieren eines bei Schritt e) erzeugten Abwassers verwendet wird.

12. Verfahren nach einem der Ansprüche 7 bis 11, wobei die Gegendruck-Dampfturbine bei einem Gegendruck von 0,6 bis 4,0 MPa betrieben wird.

13. Verfahren nach einem der Ansprüche 7 bis 12, zusätzlich umfassend die Schritte
f) Umsetzen von Erdgas oder Methan mit Wasser in Gegenwart eines Dampfreformierungskatalysators unter Wärmerückgewinnung in einem Dampferzeuger, Bereitstellen eines Reaktionsgemischs, das Wasserstoff umfasst, und
g) Abtrennen von Wasserstoff aus dem Reaktionsgemisch von Schritt f),
wobei bei Schritt f) erzeugter Dampf zum Antreiben der Gegendruck-Dampfturbine verwendet wird und bei Schritt g) abgetrennter Wasserstoff zu Schritt a) geleitet wird.

14. Verfahren nach einem der Ansprüche 7 bis 13, wobei das bei Schritt d) verwendete Lösungsmittel ausgewählt ist aus Methanol, 2-Propanol und Acetonitril und der aus der Gegendruck-Dampfturbine austretende Dampf zum Zuführen von Wärme zu einer Destillationskolonne, die das Lösungsmittel als Kopfprodukt abtrennt, verwendet wird.

## Revendications

1. Installation intégrée de fabrication d'oxyde de propène, comprenant une unité (1) de production de peroxyde d'hydrogène par un procédé à anthraquinone et une unité (2) de fabrication d'oxyde de propène à partir de propène et de peroxyde d'hydrogène, l'unité (1) de production de peroxyde d'hydrogène comprenant
a) un hydrogénateur (3) pour hydrogéner une solution de travail, contenant une alkylanthraquinone, une alkyltétrahydroanthraquinone ou les deux, avec de l'hydrogène,
b) un oxydant (4) pour oxyder une solution de travail hydrogénée avec un gaz contenant de l'oxygène, et
c) une colonne d'extraction (5) pour extraire du peroxyde d'hydrogène de la solution de travail oxydée,
et l'unité (2) de fabrication d'oxyde de propène à partir de propène et de peroxyde d'hydrogène comprenant
d) un réacteur d'époxydation (6) comportant une conduite d'alimentation (7) pour le peroxyde d'hydrogène reliée à l'unité (1) de production de peroxyde d'hydrogène et un lit fixe d'un catalyseur d'époxydation comprenant une zéolite au titane, et
e) une section de traitement (8) pour séparer de l'oxyde de propène et éventuellement du solvant d'un mélange réactionnel d'époxydation,
**caractérisée en ce que** l'installation intégrée comprend un compresseur d'air (9) entraîné par une turbine à vapeur à contre-pression (10), une conduite (11) reliant une sortie du compresseur d'air (9) à l'oxydant (4) de l'unité (1) de production de peroxyde d'hydrogène, et une conduite (12) reliant la sortie de vapeur de la turbine à vapeur à contre-pression (10) à un échangeur de chaleur (13) d'une colonne de distillation (14) de la section de traitement (8) de l'unité (2) de fabrication d'oxyde de propène.

2. Installation intégrée selon la revendication 1, dans laquelle le compresseur d'air (9) comprend une première unité de compression (15) et une deuxième unité de compression (16), ladite conduite (11) reliant une sortie du compresseur d'air (9) à l'oxydant (4) de l'unité (1) de production de peroxyde d'hydrogène est reliée à la première unité de compression (15), et une sortie de la deuxième unité de compression (16) est reliée à une entrée d'une unité de séparation d'air (17) supplémentaire.

3. Installation intégrée selon la revendication 2, comprenant une conduite (18) reliant une sortie de l'unité de séparation d'air (17) pour du gaz appauvri en oxygène à l'unité (2) de fabrication d'oxyde de propène.

4. Installation intégrée selon la revendication 2 ou 3, comprenant une conduite (19) reliant une sortie de l'unité de séparation d'air (17) pour du gaz enrichi en oxygène à une entrée de l'oxydant (4) de l'unité (1) de production de peroxyde d'hydrogène.

5. Installation intégrée selon l'une quelconque des revendications 2 à 4, dans laquelle l'unité de séparation d'air (17) comprend une unité de séparation d'air cryogénique.

6. Installation intégrée selon l'une quelconque des revendications 1 à 5, comprenant en outre une unité de reformage à la vapeur (20) pour produire de l'hydrogène à partir de méthane, l'unité de reformage à la vapeur (20) comprenant un reformeur à la vapeur (21) et un générateur de vapeur (22) chauffé par du gaz produit sortant du reformeur à la vapeur (21), une conduite (23) reliant une sortie d'hydrogène de l'unité de reformage à la vapeur (20) à une entrée de l'hydrogénateur (3) de l'unité (1) de production de peroxyde d'hydrogène, et une conduite (24) reliant une sortie de vapeur du générateur de vapeur (22) à une entrée de la turbine à vapeur à contre-pression (10).

7. Procédé intégré de fabrication d'oxyde de propène, comprenant les étapes
a) hydrogénation d'une solution de travail, contenant une alkylanthraquinone, une alkyltétrahydroanthraquinone ou les deux, avec de l'hydrogène dans un réacteur d'hydrogénation pour obtenir une solution de travail hydrogénée comprenant une alkylanthrahydroquinone, une alkyltétrahydroanthrahydroquinone ou les deux,
b) oxydation de la solution de travail hydrogénée obtenue à l'étape a) avec un gaz contenant de l'oxygène dans un réacteur d'oxydation pour obtenir une solution de travail oxydée comprenant du peroxyde d'hydrogène et une alkylanthraquinone, une alkyltétrahydroanthraquinone ou les deux,
c) extraction de peroxyde d'hydrogène de la solution de travail oxydée obtenue à l'étape b) pour obtenir une solution aqueuse de peroxyde d'hydrogène,
d) mise en réaction de propène avec la solution aqueuse de peroxyde d'hydrogène obtenue à l'étape c) en présence d'un catalyseur de zéolite au titane et d'un solvant pour fournir un mélange réactionnel comprenant de l'oxyde de propène, et
e) séparation d'oxyde de propène et de solvant du mélange réactionnel obtenu à l'étape d) à l'aide d'au moins une colonne de distillation,
**caractérisé en ce que** de l'air est comprimé avec un compresseur d'air entraîné par une turbine à vapeur à contre-pression, une partie ou la totalité de l'air comprimé est passée à l'étape b) pour fournir une partie ou la totalité dudit gaz contenant de l'oxygène, et une partie ou la totalité de la vapeur sortant de la turbine à vapeur à contre-pression est utilisée pour fournir de la chaleur à une colonne de distillation de l'étape e).

8. Procédé selon la revendication 7, dans lequel le compresseur d'air est un compresseur à deux étages, de l'air comprimé provenant de la première unité de compression est passé en tant que gaz contenant de l'oxygène à l'étape b), de l'air comprimé provenant de la deuxième unité de compression est passé à une unité de séparation d'air fournissant un flux gazeux enrichi en azote et un flux gazeux enrichi en oxygène, et la totalité ou une partie du flux gazeux enrichi en azote est passé à l'étape d) ou à l'étape e) ou aux deux étapes d) et e) pour empêcher la formation de mélanges gazeux inflammables.

9. Procédé selon la revendication 8, dans lequel la totalité ou une partie du flux gazeux enrichi en oxygène est passée à l'étape b) pour fournir du gaz contenant de l'oxygène.

10. Procédé selon la revendication 8, dans lequel la totalité ou une partie du flux gazeux enrichi en oxygène est utilisée pour oxyder un gaz d'échappement généré dans l'étape d) ou dans l'étape e) ou des gaz d'échappement des deux étapes d) et e).

11. Procédé selon la revendication 8, dans lequel la totalité ou une partie du flux gazeux enrichi en oxygène est utilisée pour oxyder une eau usée générée dans l'étape e).

12. Procédé selon l'une quelconque des revendications 7 à 11, dans lequel la turbine à vapeur à contre-pression est exploitée à une contre-pression allant de 0,6 à 4,0 MPa.

13. Procédé selon l'une quelconque des revendications 7 à 12, comprenant en outre les étapes de
f) mise en réaction de gaz naturel ou de méthane avec de l'eau en présence d'un catalyseur de reformage à la vapeur avec récupération de chaleur dans un générateur de vapeur, fournissant un mélange réactionnel comprenant de l'hydrogène, et
g) séparation d'hydrogène du mélange réactionnel de l'étape f),
dans lequel la vapeur générée à l'étape f) est utilisée pour entraîner ladite turbine à vapeur à contre-pression et l'hydrogène séparé à l'étape g) est passé à l'étape a).

14. Procédé selon l'une quelconque des revendications 7 à 13, dans lequel le solvant utilisé dans l'étape d) est choisi parmi le méthanol, le 2-propanol et l'acétonitrile et la vapeur sortant de la turbine à vapeur à contre-pression est utilisée pour fournir de la chaleur à une colonne de distillation séparant le solvant en tant que produit de tête.
